# EUROPEAN PATENT SPECIFICATION

(11) **EP 0 533 279 B1**
(45) Date of publication and mention of the grant of the patent: **06.03.1996**
(21) Application number: 92202828.7
(22) Date of filing: 15.09.1992
(51) Int. Cl.: A61K 7/30

(54) **Use of a combination of a gel and a solution, both containing an organic acid for cleaning dentures**
Verwendung von einer Kombination aus einem Gel und einer Flüssigkeit, die beide eine organische Säure enthalten zur Gebissreinigung
Utilisation d'une combinaison d'un gel et d'une solution, contenant chacun un acide organique, pour nettoyer les dentiers

(30) Priority: 16.09.1991 NL 9101559
(43) Date of publication of application: 24.03.1993
(73) Proprietor: DENTIP INTERNATIONAL B.V., NL-6040 AE Roermond (NL)
(72) Inventor: Adams, Marc Inigo, NL-6041 XH Roermond (NL)
(74) Representative: de Bruijn, Leendert C.

(56) References cited:
- WO-A-81/01969
- WO-A-81/02102
- GB-A- 543 458
- GB-A- 1 507 356
- GB-A- 1 572 032
- US-A- 1 488 315
- US-A- 3 124 506
- US-A- 4 806 173
- PATENT ABSTRACTS OF JAPAN vol. 67, (C-53)7 May 1981
- CHEMICAL ABSTRACTS, vol. 94 Columbus, Ohio, US; abstract no. 180508u, 'PHYTATES IN CLEANING AGENTS FOR DENTURES'

## Description

The invention relates to the use of a combination of a gel and a solution, both containing an organic acid for cleaning dentures.

Cleaning agents for dentures are known. Such known agents frequently contain aggressive chemicals such as hypochlorite, perborate or persulphate. A disadvantage of such chemicals is that the quality of the denture material deteriorates. As a consequence, the denture suffers discolourations and damage.

It has been found, surprisingly, that when using a combination of a gel and a solution, containing an organic acid for cleaning, such disadvantages do not arise. In this connection it is to be noted that the cleaning agents used according to the invention, i.e. the gel and the solution, are principally based on an organic acid, in particular on natural biological organic acids, but that in addition to these, additives can be present, such as surface-active substances, thickeners, aroma substances, i.e. perfume(s) and/or flavourings, and preservatives. The proviso is that the cleaning agents do not contain aggressive chemicals such as persulphate and perborate.

It is noted that European Patent Application 0360299 discloses effervescent preparations for cleaning dentures. Such preparations contain a hypochlorite-producing agent and an effervescing agent, in which is included a hypochlorite-deactivating agent. The effervescing agent consists, for example, of a mixture of an alkali metal bicarbonate and a water-soluble organic acid such as citric acid, ascorbic acid and tartaric acid. The function of such acids is thus to regulate the effervescing action.

The invention also encompasses the making-up of the single non-effervescent preparations for cleaning dentures, i.e. the gel and the solution, as well as a kit containing a combination of said preparations.

In particular, organic acids chosen from among the natural biological acids are used according to the invention.

Natural biological organic acids can be defined as organic acids occurring in nature. These are generally water-soluble.

Examples of natural biological organic acids are: acetic acid, pyruvic acid, citric acid, malic acid, (cis)aconitic acid, fumaric acid, succinic acid, (iso)citric acid, amino acids, formic acid, ascorbic acid, oxalic acid, tartaric acid, mandelic acid, ketoglutaric acid, benzoic acid, propionic acid etc.

The invention also relates to a preparation for cleaning dentures, wherein the active substance comprises at least one organic acid which is preferably chosen from among the natural, biological acids which are defined above.

In the use according to the invention, and in the preparations or kit according to the invention, the organic acid is preferably citric acid.

Compared to the known agents for cleaning dentures, the combination or kit according to the invention has at least an equally good action.

In the use and in the kit according to the invention the gel can comprise:
0.01-10% by weight, preferably 0.1-1% by weight, of organic acid,
0-20% by weight, preferably 5-10% by weight, of surface-active substance,
0-10% by weight, preferably 2-5% by weight, of thickener,
0-1% by weight, preferably 0.01-0.1% by weight, of aroma substance and
0-4% by weight, preferably 0.01-0.1% by weight, of preservative,
the remainder being water; and
the liquid can comprise:
0.01-25% by weight, preferably 8-16% by weight, of organic acid,
0-20% by weight, preferably 2-5% by weight, of surface-active substance,
0-1% by weight, preferably 0.01-0.1% by weight, of aroma substance and
0-4% by weight, preferably 0.01-0.1% by weight, of preservative,
the remainder being water.

Though citric acid is preferably used as the organic acid in the invention, other acids, such as those mentioned above, in particular tartaric acid, acetic acid and formic acid, can equally well be used.

As surface-active substances which in addition to the surface-active action possess a thickening, suspending and stabilising action there may be mentioned: anionic detergents in general, as well as fatty alcohol(ether)-sulphates and -sulphosuccinates, lauryl ether-sulphate, lauryl sulphate, alkylsulphonates and ethoxylated alkyl polyglycol ethers. Further examples are sodium lauryl-sulphate, monoethanolammonium lauryl-sulphate, sodium ethylhexyl-sulphate and lauryl fatty alcohol sulphosuccinate.

As thickeners which serve to thicken and/or to gel the preparation, there may be used electrolytes, for example inorganic salts such as sodium chloride, but also cellulose derivatives and gelatin or gelatin-like substances.

As aroma substances, there may be mentioned perfumes or eucalyptus or similar aroma substances.

As preservatives, there may be mentioned the commercially available product Euxyl/K 100, which consists of 5-chloro-2-methyl-3-(2H)-isothiazolone, 2-methyl-3(2H)-isothiazolone, together with magnesium chloride/magnesium nitrate and benzyl alcohol.

The invention also relates to a method for cleaning dentures with the use of the kit or combination as defined in the above.

The examples which follow illustrate the various possibilities of the preparations according to the invention.

### Cleaning liquid

| Formulation 1 | | | |
|---|---|---|---|
| | Minimum | Optimum | Maximum |
| citric acid | 6 % by weight | 12.0 % by weight | 25 % by weight |
| lauryl ether sulphate | 2 % by weight | 3.5 % by weight | 5 % by weight |
| sodium chloride | 2.5 % by weight | 3.5 % by weight | 5 % by weight |
| eucalyptus | 0.5 % by weight | 0.5 % by weight | 0.5 % by weight |
| Euxyl/K100 | 0.02% by weight | 0.02% by weight | 0.02% by weight |

| Formulation 2 | | | |
|---|---|---|---|
| | Minimum | Optimum | Maximum |
| tartaric acid | 6 % by weight | 12.0 % by weight | 25 % by weight |
| lauryl ether sulphate | 2 % by weight | 3.5 % by weight | 5 % by weight |
| sodium chloride | 2.5 % by weight | 3.5 % by weight | 5 % by weight |
| eucalyptus | 0.5 % by weight | 0.5 % by weight | 0.5 % by weight |
| Euxyl/K100 | 0.02% by weight | 0.02% by weight | 0.02% by weight |

| Formulation 3 | | | |
|---|---|---|---|
| | Minimum | Optimum | Maximum |
| acetic acid | 7 % by weight | 12.0 % by weight | 20 % by weight |
| lauryl ether sulphate | 2 % by weight | 3.5 % by weight | 5 % by weight |
| sodium chloride | 2.5 % by weight | 3.5 % by weight | 5 % by weight |
| eucalyptus | 0.5 % by weight | 0.5 % by weight | 0.5 % by weight |
| Euxyl/K100 | 0.02% by weight | 0.02% by weight | 0.02% by weight |

| Formulation 4 | | | |
|---|---|---|---|
| | Minimum | Optimum | Maximum |
| formic acid | 6 % by weight | 12.0 % by weight | 25 % by weight |
| lauryl ether sulphate | 2 % by weight | 3.5 % by weight | 5 % by weight |
| sodium chloride | 2.5 % by weight | 3.5 % by weight | 5 % by weight |
| eucalyptus | 0.5 % by weight | 0.5 % by weight | 0.5 % by weight |
| Euxyl/K100 | 0.02% by weight | 0.02% by weight | 0.02% by weight |

### Cleaning gel

| Formulation 5 | | | |
|---|---|---|---|
| citric acid | 0.4 % by weight | 0.6 % by weight | 2.0 % by weight |
| lauryl ether sulphate | 8.0 % by weight | 11.0 % by weight | 15.0 % by weight |
| thickener/common salt | 2.5 % by weight | 3.5 % by weight | 3.5 % by weight |
| perfume/eucalyptus | 0.05% by weight | 0.05% by weight | 0.05% by weight |
| Euxyl/K100 | 0.02% by weight | 0.02% by weight | 0.02% by weight |

| Formulation 6 | | | |
|---|---|---|---|
| tartaric acid | 0.4 % by weight | 0.6 % by weight | 2.0 % by weight |
| lauryl ether sulphate | 8.0 % by weight | 11.0 % by weight | 15.0 % by weight |
| thickener/common salt | 2.5 % by weight | 3.5 % by weight | 3.5 % by weight |
| perfume/eucalyptus | 0.05% by weight | 0.05% by weight | 0.05% by weight |
| Euxyl/K100 | 0.02% by weight | 0.02% by weight | 0.02% by weight |

| Formulation 7 | | | |
|---|---|---|---|
| acetic acid | 0.4 % by weight | 0.6 % by weight | 2.0 % by weight |
| lauryl ether sulphate | 8.0 % by weight | 11.0 % by weight | 15.0 % by weight |
| thickener/common salt | 2.5 % by weight | 3.5 % by weight | 3.5 % by weight |
| perfume/eucalyptus | 0.05% by weight | 0.05% by weight | 0.05% by weight |
| Euxyl/K100 | 0.02% by weight | 0.02% by weight | 0.02% by weight |

| Formulation 8 | | | |
|---|---|---|---|
| formic acid | 0.4 % by weight | 0.6 % by weight | 2.0 % by weight |
| lauryl ether sulphate | 8.0 % by weight | 11.0 % by weight | 15.0 % by weight |
| thickener/common salt | 2.5 % by weight | 3.5 % by weight | 3.5 % by weight |
| perfume/eucalyptus | 0.05% by weight | 0.05% by weight | 0.05% by weight |
| Euxyl/K100 | 0.02% by weight | 0.02% by weight | 0.02% by weight |

- Minimum: = the first detectible cleaning effect
- Optimum: = the optimum cleaning effect without damage occurring to the denture material
- Maximum: = the limit, at which damage to the denture material by the active substance of the preparation can no longer be ruled out.

The effectiveness of the preparations according to the invention was determined in accordance with the following Instruction:

### Instruction

### Soiling of dentures

The dentures were soiled with the following products: red wine, tartar, coffee, tea, nicotine and lipstick.

Prepare a saturated solution of tartar and add sufficient red wine, strong coffee and strong tea, so that the denture can be entirely submerged therein. Put the denture into this solution for 10 minutes and let the denture dry at a temperature of 40°C. Blow cigarette smoke over the denture for 15 minutes and thereafter smear the denture with red lipstick. Repeat this procedure five times.

### Cleaning of the dentures with gel

The denture is cleaned by means of a denture brush and the gel, as prescribed.

### Cleaning of the dentures with the aid of a liquid preparation

The denture is placed for 8 hours in a solution, diluted 1:4, of the preparation.

### Assessment of cleaning

After cleaning, the denture is inspected visually with attention to colour, smoothness and odour.

It was found that the non-effervescent preparations according to the invention, which are described above, possess an excellent cleaning action in tests according to the Instruction. This action is at least as good as that of preparations according to the state of the art.

## Claims

1. Use of a combination of a gel and a solution, both containing an organic acid for cleaning dentures.

2. Use according to Claim 1, wherein the organic acid is chosen from among the natural biological organic acids, in particular citric acid.

3. Use according to Claims 1 or 2 wherein the gel comprises:
0.01-10% by weight, preferably 0.1-1% by weight, of organic acid,
0-20% by weight, preferably 5-10% by weight, of surface-active substance,
0-10% by weight, preferably 2-5% by weight, of thickener,
0-1% by weight, preferably 0.01-0.1% by weight, of aroma substance,
0-4% by weight, preferably 0.01-0.1% by weight, of preservative;
and further water; and the liquid comprises:
0.01-25% by weight, preferably 8-16% by weight, of organic acid,
0-20% by weight, preferably 2-5% by weight, of surface-active substance,
0-1% by weight, preferably 0.01-0.1% by weight, of aroma substance,
0-4% by weight, preferably 0.01-0.1% by weight, of preservative, and further water.

4. Kit for cleaning dentures at least comprising a gel and a solution both containing an organic acid.

5. Kit according to Claim 4, wherein the organic acid is chosen from among the natural biological organic acids, in particular citric acid.

6. Kit according to Claim 4 or 5, wherein the gel comprises:
0.01-10% by weight, preferably 0.1-1% by weight, of organic acid,
0-20% by weight, preferably 5-10% by weight, of surface-active substance,
0-10% by weight, preferably 2-5% by weight, of thickener,
0-1% by weight, preferably 0.01-0.1% by weight, of aroma substance,
0-4% by weight, preferably 0.01-0.1% by weight, of preservative;
and further water; and the liquid comprises:
0.01-25% by weight, preferably 8-16% by weight, of organic acid,
0-20% by weight, preferably 2-5% by weight, of surface-active substance,
0-1% by weight, preferably 0.01-0.1% by weight, of aroma substance,
0-4% by weight, preferably 0.01-0.1% by weight, of preservative, and further water.

7. A method for cleaning dentures using the kit according to any of Claims 4-6.

## Patentansprüche

1. Verwendung einer Kombination aus einem Gel und einer Losung, die beide eine organische Säure enthalten, zur Gebissreinigung.

2. Verwendung nach Anspruch 1, bei dem man als organische Säure eine natürlich vorkommende, biologische organische Säure, insbesondere Zitronensäure, auswählt.

3. Verwendung nach Anspruch 1 oder 2, bei dem das Gel 0,01-10 Gew.% - vorzugsweise 0,1-1 Gew.% - organische Säure, 0-20 Gew.% - vorzugsweise 5-10 Gew.% - oberflächenaktive Substanz, 0-10 Gew.% - vorzugsweise 2-5 Gew.% - Verdickungsmittel, 0-1 Gew.% - vorzugsweise 0,01-0,1 Gew.% - Aromastoff, 0-4 Gew.% - vorzugsweise 0,01-0,1 Gew.% - Konservierungsmittel, sowie Wasser umfasst; und
die Flüssigkeit 0,01-25 Gew.% - vorzugsweise 8-16 Gew.% - organische Säure, 0-20 Gew.% - vorzugsweise 2-5 Gew.% - oberflächenaktive Substanz, 0-1 Gew.% - vorzugsweise 0,01-0,1 Gew.% - Aromastoff, 0-4 Gew.% - vorzugsweise 0,01-0,1 Gew.% - Konservierungsmittel, sowie Wasser umfasst.

4. Set zur Gebissreinigung, das mindestens ein Gel und eine Lösung umfasst, die beide eine organische Säure enthalten.

5. Set nach Anspruch 4, bei dem man als organische Säure eine natürlich vorkommende, biologische organische Säure, insbesondere Zitronensäure, auswählt.

6. Set nach Anspruch 4 oder 5, bei dem das Gel 0,01-10 Gew.% - vorzugsweise 0,1-1 Gew.% - organische Säure, 0-20 Gew.% - vorzugsweise 5-10 Gew.% - oberflächenaktive Substanz, 0-10 Gew.% - vorzugsweise 2-5 Gew.% - Verdickungsmittel, 0-1 Gew.% - vorzugsweise 0,01-0,1 Gew.% - Aromastoff, 0-4 Gew.% - vorzugsweise 0,01-0,1 Gew.% - Konservierungsmittel, sowie Wasser umfasst; und
die Flüssigkeit 0,01-25 Gew.% - vorzugsweise 8-16 Gew.% - organische Säure, 0-20 Gew.% - vorzugsweise 2-5 Gew.% - oberflächenaktive Substanz, 0-1 Gew.% - vorzugsweise 0,01-0,1 Gew.% - Aromastoff, 0-4 Gew.% - vorzugsweise 0,01-0,1 Gew.% - Konservierungsmittel, sowie Wasser umfasst.

7. Verfahren zur Gebissreinigung, bei dem man das Set gemäss einem der Ansprüche 4-6 verwendet.

## Revendications

1. Utilisation d'une combinaison d'un gel et d'une solution, contenant chacun un acide organique pour le nettoyage de dentiers.

2. Utilisation selon la revendication 1, dans laquelle l'acide organique est choisi parmi les acides organiques biologiques naturels, en particulier l'acide citrique.

3. Utilisation selon la revendication 1 ou 2, dans laquelle le gel comprend :
0,01-10 % en poids, de préférence 0,1-1 % en poids d'acide organique,
0-20 % en poids, de préférence 5-10 % en poids d'une substance tensioactive,
0-10 % en poids, de préférence 2-5 % en poids d'un épaississant,
0-1 % en poids, de préférence 0,01-0,1 % en poids d'une substance aromatisante,
0-4 % en poids, de préférence 0,01-0,1 % en poids d'un conservant,
et en outre de l'eau; et le liquide comprend :
0,01-25 % en poids, de préférence 8-16 % en poids d'acide organique,
0-20 % en poids, de préférence 2-5 % en poids d'une substance tensioactive,
0-1 % en poids, de préférence 0,01-0,1 % en poids d'une substance aromatisante,
0-4 % en poids, de préférence 0,01-0,1 % en poids d'un conservant, et en outre de l'eau.

4. Nécessaire pour le nettoyage des dentiers, comprenant au moins un gel et une solution contenant chacun un acide organique.

5. Nécessaire selon la revendication 4, dans lequel l'acide organique est choisi parmi les acides organiques biologiques naturels, en particulier l'acide citrique.

6. Nécessaire selon la revendication 4 ou 5, dans lequel le gel comprend :
0,01-10 % en poids, de préférence 0,1-1 % en poids d'acide organique,
0-20 % en poids, de préférence 5-10 % en poids d'une substance tensioactive,
0-10 % en poids, de préférence 2-5 % en poids d'un épaississant,
0-1 % en poids, de préférence 0,01-0,1 % en poids d'une substance aromatisante,
0-4 % en poids, de préférence 0,01-0,1 % en poids d'un conservant,
et en outre de l'eau; et le liquide comprend :
0,01-25 % en poids, de préférence 8-16 % en poids d'acide organique,
0-20 % en poids, de préférence 2-5 % en poids d'une substance tensioactive,
0-1 % en poids, de préférence 0,01-0,1 % en poids d'une substance aromatisante,
0-4 % en poids, de préférence 0,01-0,1 % en poids d'un conservant, et en outre de l'eau.

7. Procédé pour le nettoyage des dentiers utilisant le nécessaire selon l'une quelconque des revendications 4-6.
